(19) Europäisches Patentamt
European Patent Office
Office européen des brevets

(11) Publication number: **0 235 877**
**A1**

# (12) EUROPEAN PATENT APPLICATION

(21) Application number: **87300106.9**

(22) Date of filing: **07.01.87**

(51) Int. Cl.⁴: **C 07 D 249/08**, A 01 N 43/653

(30) Priority: **28.01.86 GB 8601952**

(43) Date of publication of application: **09.09.87 Bulletin 87/37**

(84) Designated Contracting States: **AT BE DE ES FR GB IT LU NL SE**

(71) Applicant: **IMPERIAL CHEMICAL INDUSTRIES PLC, Imperial Chemical House Millbank, London SW1P 3JF (GB)**

(72) Inventor: **Griffin, David Alan, 12 Orion Roman Hill, Bracknell Berkshire (GB)**

(74) Representative: **Ricks, Michael James et al, Imperial Chemical Industries PLC Legal Department: Patents PO Box 6 Bessemer Road, Welwyn Garden City Herts, AL7 1HD (GB)**

## (54) Triazolyl butanoic acid derivatives, process for their preparation and their use as plant growth regulators.

(57) A plant growth regulating composition comprises a triazole derivative having the general formula (I) and a carrier or diluent,

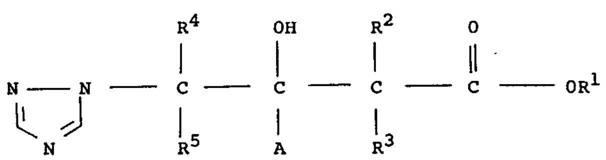

wherein $R^1$ is an alkyl group containing from 1 to 4 carbon atoms; $R^2$ and $R^3$, which may be the same or different, are each separately an alkyl group containing from 1 to 4 carbon atoms, or $R^2$ and $R^3$, together with the carbon atom joining them, form a cycloalkyl group containing from 3 to 6 carbon atoms; $R^4$ and $R^5$, which may be the same or different, each represent hydrogen or an alkyl group containing from 1 to 4 carbon atoms; and A represents an optionally substituted phenyl group; and acid addition salts, ethers, esters and metal complexes thereof. Certain compounds of formula (I) are novel.

EP 0 235 877 A1

TITLE MODIFIED
see front page



0 235 877

HETEROCYCLIC COMPOUNDS

This invention relates to triazole compounds useful as plant growth regulators, to a process for preparing them, to plant growth regulating compositions containing them, and to methods of using them to regulate plant growth.

In EP 0046633 A there are described compounds having fungicidal and plant growth regulating activity and having the general formula (I) :

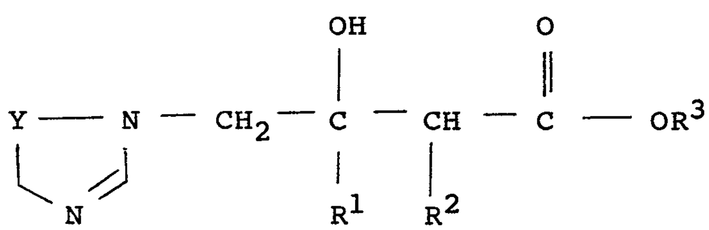

Formula (I)

and stereoisomers thereof, wherein Y is -CH= or =N-, $R^1$ is alkyl, cycloalkyl or optionally substituted phenyl and $R^2$ and $R^3$, which may be the same or different, are hydrogen, alkyl, cycloalkyl, (eg, cyclopropyl, cyclopentyl or cyclohexyl), optionally substituted phenyl or optionally substituted benzyl, or together form a lactone ring; and acid addition salts, ethers, esters and metal complexes thereof.

In European Patent Publication No 106,515 there are described compounds useful as human and argicultural fungicides and having the general Formula (A):-

0 235 877

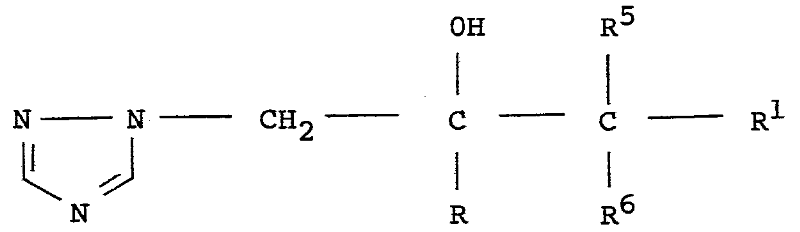

(A)

wherein R is phenyl optionally substituted by 1 to 3 substituents each independently selected from F, Cl, Br, $CF_3$, $C_1$ to $C_4$ alkyl and $C_1$ to $C_4$ alkoxy, or R is a 5-chloropyrid-2-yl group; $R^1$ is -CN, $-CSNH_2$ or $-CONR^2R^3$ where the values of $R^2$ and $R^3$ are defined; and $R^5$ and $R^6$ are H or methyl.

As intermediates in the preparation of compounds of Formula (A), EP 106, 515 discloses compounds having the general Formula (B):-

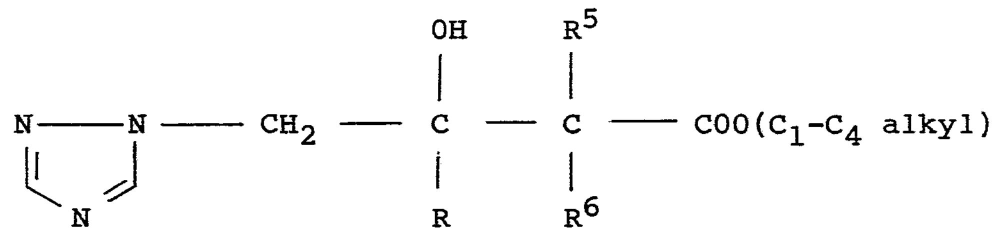

(B)

where R, $R^5$ and $R^6$ have the meanings given in respect of Formula A.

There is no indication that these intermediates have any form of activity in their own right.

According to the present invention there is provided a plant growth regulating composition comprising a triazole derivative having the general Formula (I) and stereoisomers thereof and a carrier or diluent:-

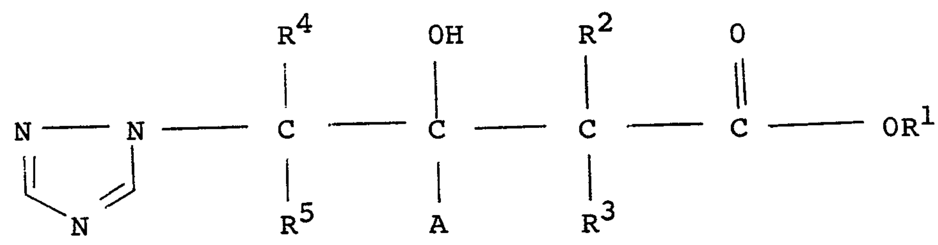

(I)

wherein $R^1$ is an alkyl group containing from 1 to 4 carbon atoms; $R^2$ and $R^3$, which may be the same or different, are each separately an alkyl group containing from 1 to 4 carbon atoms or $R^2$ and $R^3$, together with the carbon atom joining them, form a cycloalkyl group containing from 3 to 6 carbon atoms; $R^4$ and $R^5$, which may be the same or different, each represent hydrogen or an alkyl group containing from 1 to 4 carbon atoms; and A represents an optionally substituted phenyl group; and acid addition salts, ethers, esters and metal complexes thereof.

The compounds of the invention may contain chiral centres. Such compounds are generally obtained in the form of racemic mixtures. However, these and other mixtures can be separated into the individual isomers by methods known in the art, and this invention embraces such isomers.

The present invention includes salts, esters and metal complexes of the compounds of general Formula (I). As examples of esters there may be mentioned for example acetates or benzoates. Without limitation of the generality of the above statement, the present invention also includes any compound which breaks down in agrochemical use to form a compound of general Formula (I).

TABLE I

| COMPOUND NO | $R^1$ | $R^2$ | $R^3$ | $R^4$ | $R^5$ | A | Melting Point °C |
|---|---|---|---|---|---|---|---|
| 1 | $-CH_3$ | $-CH_3$ | $-CH_3$ | H | H | 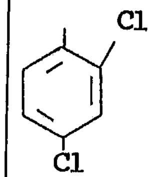 | 118.5-119.5 |
| 2 | $-CH_3$ | $-C_2H_5$ | $-C_2H_5$ | H | H | 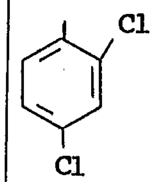 | 96-97 |
| 3 | $-C_2H_5$ | $-C_2H_5$ | $-C_2H_5$ | H | H | 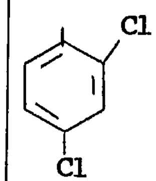 | 116-117 |
| 4 | $-CH_3$ | 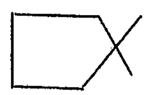 | | H | H | 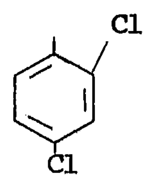 | 114-115 |

TABLE I CONTINUED

| COMPOUND NO | $R^1$ | $R^2$ | $R^3$ | $R^4$ | $R^5$ | A | Melting Point °C |
|---|---|---|---|---|---|---|---|
| 5 | $-C_2H_5$ | 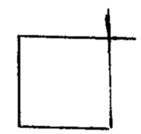 | | H | H | 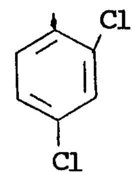 | 100-102 |
| 6 | $-CH_3$ | 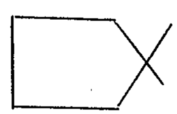 | | H | H | 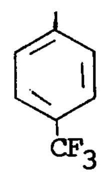 | GUM |
| 7 | $-CH_3$ | $-CH_3$ | $-CH_3$ | H | H | 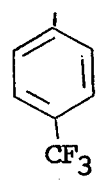 | 98-99 |
| 8 | $CH_3$ | $CH_3$ | $C_2H_5$ | H | H | 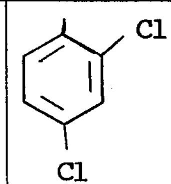 | 70.5-71.5 |

$R^1$ is preferably methyl, ethyl or propyl (iso or normal).

$R^2$ and $R^3$ maybe the same and are preferably both methyl or ethyl or are separately methyl or ethyl. When $R^2$ and $R^3$, together with the carbon atom joining them, form a cycloalkyl group, the cycloalkyl group is preferably cyclobutyl or cyclopentyl.

$R^4$ and $R^5$ are preferably hydrogen or methyl.

The group A may be phenyl or phenyl substituted by, for example one or more of halogen, trifluoromethyl, or alkyl (for example alkyl containing from 1 to 4 carbon atoms). Preferably A is trifluoromethyl phenyl or halophenyl having one, two or three halogen atoms as substitutents including Cl, F, Br, or I. Preferably these substituent(s) are located at the 4-position or 2,4-positions. Chlorophenyl, and in particular dichlorophenyl, for example 2,4-dichlorophenyl is especially preferred as is 4-trifluoromethylphenyl.

In an especially preferred compound, $R^1$ is methyl, $R^2$ and $R^3$ are both methyl, $R^4$ and $R^5$ are both hydrogen and A is 2,4-dichlorophenyl.

According to a further aspect of the present invention, there is provided a triazole derivative of formula (I)

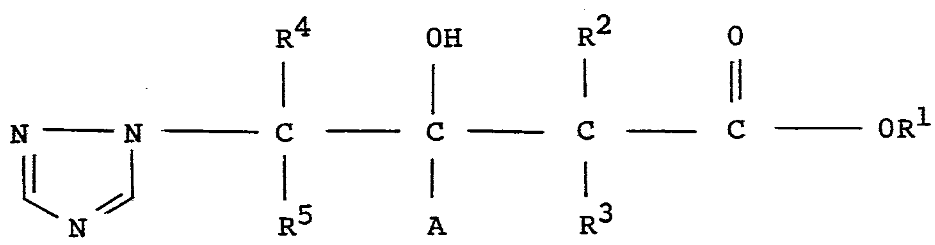

(I)

and stereoisomrs thereof, wherein $R^1$ is an alkyl group containing from 1 to 4 carbon atoms; $R^2$ is an alkyl group

containing from 1 to 4 carbon atoms, $R^3$ is an alkyl group containing from 2 to 4 carbon atoms, or $R^2$ and $R^3$, together with the carbon atom joining them, form a cycloalkyl group containing from 3 to 6 carbon atoms; $R^4$ and $R^5$, which may be the same or different, each represent hydrogen or an alkyl group containing from 1 to 4 carbon atoms; and A represents an optionally substituted phenyl group; and acid addition salts, ethers, esters and metal complexes thereof.

Preferred values of A, $R^1$, $R^2$, $R^3$, $R^4$ and $R^5$ are as indicated above. Compounds of the invention and for use in the plant growth regulating composition of the invention and having the formula (I) are shown in Table I which lists the values of $R^1$, $R^2$, $R^3$, $R^4$, $R^5$ and A. Where a cycloalkyl structure is listed spanning both the $R^2$ and $R^3$ columns (Compound Numbers 4, 5 and 6), this indicates that $R^2$ and $R^3$, together with the carbon atom joining them, form the represented cycloalkyl group.

The compounds of general Formula (I) may be produced by reacting a compound of general Formula (II) (wherein $R^4$ and $R^5$ and A have the meanings given previously) with a compound of general Formula (III) (wherein $R^1$, $R^2$ and $R^3$ have the meanings given previously and Z is an alkyl or aryl group) in the presence of a Lewis acid such as titanium tetrachloride:-

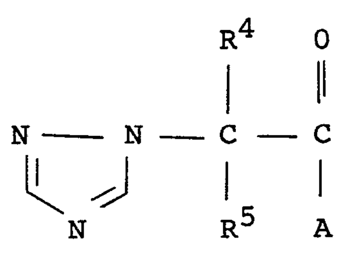

(II)

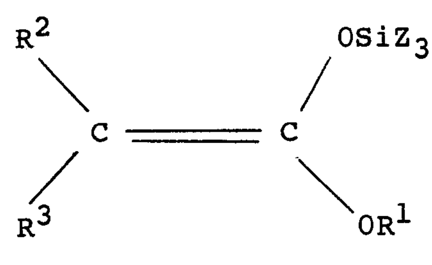

(III)

The reaction conveniently takes place in a suitable solvent and under an inert atmosphere. Z is preferably a lower alkyl group, for example a methyl group. The product may be isolated by conventional means.

The compounds of general Formula (II) and (III) may be made by methods set out in the literature: for example JCS Chem Commun, 1971, 136; J Organomet. Chem, 46, 59, (1972); Synthesis, 9, 723, (1982); Zh. Obshch. Khim, 1979, 2753.

The salts, ethers, esters and metal complexes of the compounds of general Formula (I) can be prepared from the latter in known manner. For example the complexes may be made by reacting the uncomplexed compound with a metal salt in a suitable solvent.

The plant growth regulating effects of the compounds are manifested as, for example, by a stunting or dwarfing effect on the vegetative growth of woody and herbaceous mono- and di-cotyledonous plants. Such stunting or dwarfing may be useful, for example, in peanuts, cereals (such as wheat, barley and rice), oil seed rape, field beans, sunflowers, potatoes and soya bean where reduction in stem height, with or without further advantageous effects such as stem strengthening, thickening and shortening, internode shortening, increased buttress root formation and more erect stem and leaf orientation, may reduce the risk of lodging and may also permit increased amounts of fertiliser to be applied. The stunting of woody species is useful in controlling of the growth of trees under power lines etc.

The growth of trees acting as windbreaks, for example in orchards, may be controlled to prevent the need for excessive cutting back of foliage. Control of the growth of conifers may be useful in plantation management.

Compounds which induce stunting or dwarfing may also be useful in modifying the stem growth of sugar cane thereby increasing the concentration of sugar in the cane

at harvest; in sugar cane, the flowering and ripening may be controllable by applying the compounds. Stunting of peanuts can assist in harvesting.

Growth retardation of grasses can help maintenance of grass swards. Examples of suitable grasses are Strenotaphrum secundatum (St. Augustine grass), Cynosurus cristatus, Lolium multiflorum and perenne, Agrostis tenuis, Cynodon dactylon (Bermuda grass), Dactylisglomerata, Festuca spp. (eg, Festuca rubra) and Poa spp. (eg, Poa pratense). The compounds may stunt grasses without significant phytotoxic effects and without deleteriously affecting the appearance (particularly the colour) of the grass; this makes such compounds attractive for use on ornamental lawns and on grass verges. They may also have an effect on flower head emergence in, for example, grasses.

The compounds can also stunt weed species present in the grasses; examples of such weed species are sedges (eg, Cyperus spp.) and dicotyledonous weeds (eg, daisy, plantain, knotweed, speedwell, thistle, docks and ragwort). The growth of non-crop vegetation (eg, weeds or cover vegetation) can be retarded thus assisting in the maintenance of plantation and field crops.

In fruit orchards, particularly orchards subject to soil erosion, the presence of grassover is important. However excessive grass growth requires substantial maintenance. The compounds of the invention could be useful in this situation as they could restrict growth without killing the plants which would lead to soil erosion; at the same time the degree of competition for nutrients and water by the grass would be reduced and this could result in an increased yield of fruit. In some cases, one grass species may be stunted more than another grass species; this selectivity could be useful, for example, for improving the quality of a sward by preferential suppression of the

growth of undesirable species.

The dwarfing may also be useful in miniaturising ornamental, household, garden and nursery plants (eg, poinsettias, roses, chrysanthemums, carnations, tulips and daffodils).

As indicated above, the compounds can also be used to stunt woody species. This property can be used to control hedgerows or to shape or reduce the need for pruning, of fruit trees (eg, apples, pears, cherries, peaches, vines etc). Some coniferous trees are not significantly stunted by the compounds so the compounds could be useful in controlling undesirable vegetation in conifer nurseries.

The plant growth regulating effect may (as implied above) manifest itself in an increase in crop yield; or in an ability in orchards and other crops to increase fruit set, pod set and grain set.

In the potato, vine control in the field and inhibition of sprouting in the store may be possible.

Other plant growth regulating effects caused by the compounds include alteration of leaf angle and changes in leaf morphology (both of which may permit increased light interception and utilization) and promotion of tillering in monocotyledonous plants. Improved light interception is of value in all major world crops, eg, wheat, barley, rice, maize, soya, sugarbeet, potatoes, plantation crops and orchard crops. The leaf angle effect may be useful for example in altering the leaf orientation of, for example, potato crops thereby letting more light into the crops and inducing an increase in photosynthesis and tuber weight. By increasing tillering in monocotyledonous crops (eg, rice), the number of flowering shoots per unit area may be increased thereby increasing the overall grain yield of such crops. In addition better control and modification of hierarchical relationships is

possible both in vegetative and reproductive stages of monocotyledonous and dicotyledenous plant growth, especially in cereals such as wheat, barley, rice and maize, whereby the number of flowering shoots per unit area may be increased and the size distribution of grains within the ear may be modified in such a way as to increase yield.

In the treatment of rice plants, or rice crops the invention compounds can be applied, eg, as granules or a granular formulation, for example as slow release granules, to nursery boxes, paddy water and other like cultivation loci and media. Paddy rice may be treated by submerged application of granules. In grass swards, especially amenity grass, an increase in tillering could lead to a denser sward which may result in increased resilience in wear; and to increased yields and better quality of forage grass, eg, improved digestibility and palatability.

The treatment of plants with the compounds can lead to the leaves developing a darker green colour. In dicotyledonous plants such as soyabean and cotton, there may be promotion of sideshooting.

The compounds may inhibit, or at least delay, the flowering of sugar beet (and thereby may increase sugar yield) or otherwise modify the flowering patterns in many other crops. They may also reduce the size of sugar beet without reducing significantly the sugar yield thereby enabling an increase in planting density to be made. Similarly in other root crops (eg, turnip, swede, mangold, parsnip, beetroot, yam and cassava) it may be possible to increase the planting density.

The compounds could be useful in restricting the vegetative growth of cotton thereby leading to an increase in cotton yield. Crop yields may also be increased by improvement of the harvest index (ie, the harvested yield as a proportion of the total dry matter produced) by altering dry matter partitioning. This

applies to all the aforementioned root, pod, cereal, tree, plantation and orchard crops.

The compounds may be useful in rendering plants resistant to stress since the compounds can delay the emergence of plants grown from seed, shorten stem height and delay flowering; these properties could be useful in preventing frost damage in countries where there is significant snow cover in the winter since then the treated plants would remain below snow cover during the cold weather. Further the compounds may cause drought or cold resistance in certain plants.

When applied as seed treatments at low rates the compounds may have a growth stimulating effect on plants.

It is to be understood that the compounds of the present invention will not necessarily show all the above mentioned plant growth regulating effects. The compounds of the present invention are generally very effective as retardants on a range of monocotyledinous and dicotyledinous species, and generally show excellent reduction of interligular length and overall height. Interligular length reduction is an indication of internode reduction in mature plants and, together with overall height reduction consequently limits the susceptibility of the plants to lodging. This effect is most pronounced on cereals such as spring barley, and in particular on rice. Thus on rice the compounds are very effective at exceptionally low rates of application. On spring barley the rate response is different to that on rice, and higher rates of appliccation are required for an effective response. The addition of a complementary plant growth regulator, for example a quaternary ammonium compound such as chlormequat chloride, may assist in providing a more balanced rate response on spring barley. The compounds of the invention generally show a substantial green-up effect

associated with their retardant activity.

In carrying out the plant growth regulating method of the invention, the amount of compound to be applied to regulate the growth of plants will depend upon a number of factors, for example the particular compound selected for use, and the identity of the plant species whose growth is to be regulated. In general an application rate of 0.01 to 15, for example from 0.1 to 5, kg per hectare is effective. However, highly active compounds, for example the compound of Example 1 as applied to rice, may show activity even at rates below 0.01 kg per hectare, for example as low as 0.001 kg/hectare. With the use of biodegradable polymeric slow release granules rates of 1 to 10 g per hectare are feasible, whilst electrodynamic spraying techniques may also deploy lower rates of application.

It should be noted that on certain plants (and especially on dicotyledenous plants) application rates within the higher end of the above ranges may give rise to undesired phytotoxic effects. The acceptable level of such undesired effects will depend on the plant species concerned and the specific plant growth regulating effect it is desired to achieve. In general however highly active compounds which are effective at low rates of application are preferred as undesired phytotoxic effects are rarely of any significance at low application rates. Routine tests may be necessary to determine the best rate of application of a specific compound for any specific purpose for which it is suitable.

The compounds may be used as such for plant growth regulating purposes but are more conveniently formulated into compositions for such usage. The invention thus provides a plant growth regulating composition comprising a compound of general formula (I) as hereinbefore defined, or a salt, esteror metal complex thereof; and, optionally, a carrier or diluent.

The invention also provides a method of regulating plant growth, which comprises applying to the plant, to seed of a plant or to the locus of a plant or seed, a compound, or a salt, ester or metal complex thereof, as hereinbefore defined, or a composition containing the same.

The compounds, salts metal complexes, and esters can be applied in a number of ways, for example they can be applied, formulated or unformulated, directly to the foliage of a plant, or they can be applied also to bushes and trees, to seeds or to other medium in which plants, bushes or trees are growing or are to be planted, or they can be sprayed on, dusted on or applied as a cream or paste formulation, or they can be applied as a vapour; or as slow release granules. Application can be to any part of the plant, bush or tree, for example to the foliage, stems, branches or roots, or to soil surrounding the roots, or to the seed before it is planted; or to the soil generally, to paddy water or to hydroponic culture systems. The invention compounds may also be injected into plants or trees and they may also be sprayed onto vegetation using electrodynamic spraying techniques.

The term "plant" as used herein includes seedlings, bushes and trees.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, attapulgite, bentonite, kieselguhr, dolomite, calcium carbonate, talc, powdered magnesia, Fuller's earth, gypsum, Hewitt's earth, diatomaceous earth and China clay. Such granules can be preformed granules suitable for application

to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide), or using an aqueous suspension or emulsion with a finely divided active ingredient.

The compositions of the present invention may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (eg, 2-ethoxyethanol and 2-butoxyethanol).

The compositions to be used as sprays may also be in the form of aerosols wherein the formulation is held in a container under pressure in the presence of a propellant, eg, fluorotrichloromethane or dichlorodifluoromethane or a hydrocarbon (eg, butane)/water emulsion.

The compounds can be mixed in the dry state with a pyrotechnic mixture to form a composition suitable for generating in enclosed spaces a smoke containing the

compounds.

Alternatively, the compounds may be used in a micro-encapsulated form. They may also be formulated in biodegradable polymeric formulations to obtain a slow, controlled release of the active substance.

By including suitable additives, for example additives for improving the distribution, adhesive power and resistance to rain on treated surfaces, the different compositions can be better adapted for various utilities.

The compounds can be used as mixtures with fertilisers (eg, nitrogen-, potassium- or phosphorus-containing fertilisers). Compositions comprising only granules of fertiliser incorporating, for example coated with, the compound are preferred. Such granules suitably contain up to 25% by weight of the compound. The invention therefore also provides a fertiliser composition comprising the compound of general formula (I) or a salt, ester or metal complex thereof.

The compositions may also be in the form of liquid preparations for use as dips or sprays which are generally aqueous dispersions or emulsions containing the active ingredient in the presence of one or more surfactants eg, wetting agent(s), dispersing agent(s), emulsifying agent(s) or suspending agent(s); or which are spray formulations of the kind suitable for use in electrodynamic spraying techniques. The foregoing agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or ammonium lignosulphonate, butylnaphthalene sulphonate, and a mixture of sodium diisopropyl- and triisopropylnaphthalene sulphonates).

Suitable non-ionic agents are the condensation products of ethylene oxide with fatty alcohols such as oleyl or cetyl alcohol, or with alkyl phenols such as octyl- or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), and the concentrate is to be diluted with water before use. These concentrates often should be able to withstand storage for prolonged periods and after such storage be capable of dilution with water in order to form aqueous preparations which remain homogeneous for a sufficient time to enable them to be applied by conventional and electrodynamic spray equipment. The concentrates may conveniently contain up to 95%, suitably 10-85%, for example 25-60%, by weight of the active ingredient(s). These concentrates suitably contain organic acids (eg, alkaryl or aryl sulphonic acids such as xylenesulphonic acid or dodecyl benzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient(s) depending upon the intended purpose, but an aqueous preparation containing

0.0005% to 10%, or 0.01% to 10%, by weight of active ingredient(s) may be used.

The compositions of this invention can comprise also one or more additional compound(s) having biological activity, eg, compounds having similar or complementary fungicidal or plant growth activity or compounds having, herbicidal or insecticidal activity.

The additional fungicidal compound can be, for example, one which is capable of combating ear diseases of cereals (eg, wheat) such as Septoria, Gibberella and Helminthosporium spp., seed and soil borne diseases and downy and powdery mildews on grapes and powdery mildew and scab on apple etc. Examples of suitable additional fungicidal compound are imazalil, benomyl, carbendazim, thiophanate-methyl, captafol, captan, sulphur, triforine, dodemorph, tridemorph, pyrazophos, furalaxyl, ethirimol, tecnazene, dimethirimol, bupirimate, chlorothalonil, vinclozolin, procymidone, iprodione, metalaxyl, forsetyl-aluminium, carboxin, oxycarboxin, fenarimol, nuarimol, fenfuram, methfuroxan, nitrotal-isopropyl, triadimefon, thiabendazole, etridiazole, triadimenol, biloxazol, dithianon, binapacryl, quinomethionate, guazatine, dodine fentin acetate, fentin hydroxide, dinocap, folpet, dichlofluanid, ditalimphos, kitazin, cycloheximide, dichlobutrazol, a dithiocarbamate, prochlorez, a copper compound, a mercury compound, 1-(2-cyano-2-methoxyiminoacetyl)-3-ethyl urea, fenaponil, ofurace, propiconazole, etaconazole and fenpropemorph and fenpropidine.

The compounds of general formula (I) can be mixed with soil, peat or other rooting media for the protection of plants against seed-borne, soil-borne or foliar fungal diseases.

Suitable additional insecticides are Pirimor, Croneton, dimethoate, Metasystox, pyrethroid insecticides

and formothion.

The other, additional, plant growth regulating compound can be one which controls weeds or seedhead formation, improves the level or longevity of the plant growth regulating activity of the compounds of general formula (I), selectively controls the growth of the less desirable plants (eg, grasses) or causes the compound of general formula (I) to act faster or slower as a plant growth regulating agent. Some of these other agents will also be herbicides.

Examples of suitable plant growth regulating compounds, which can display synergy in admixture, or use, with the invention compounds are the gibberellins (eg, $GA_3$, $GA_4$ and $GA_7$), the auxins (eg, indoleacetic acid, indolebutyric acid, naphthoxyacetic acid or naphthylacetic acid), the cytokinins (eg, kinetin, diphenylurea, benzimidazole, benzyladenine or benzylaminopurine), phenoxyacetic acids (eg, 2,4-D or MCPA), substituted benzoic acids (eg, triiodobenzoic acid), morphactins (eg, chlorfluorecol), maleic hydrazide, glyphosate, glyphosine, long chain fatty alcohols and acids, dikegulac, fluoridamid, mefluidide, substituted quaternary ammonium and phosphonium compounds (eg, chlormequat* chlorphonium, phosphonl D or mepiquat*), ethephon, carbetamide, methyl-3,6-dichloroanisate, daminozide*, asulam, abscisic acid, isopyrimol, 1-(4-chlorophenyl)-4,6-dimethyl-2-oxo-1,2-dihydropyridine-3-carboxylic acid, hydroxybenzonitriles (eg, bromoxynil), difenzoquat*, benzoylprop-ethyl 3,6-dichloropicolinic acid, uniconazole, triapenthenol, flurprimidol, paclobutrazol, tetcyclacis tecnazene and amidichlor. Synergy will be most likely to occur with those of the foregoing which are quaternary ammonium compounds and with those marked with an asterisk.

For certain applications, for example in the injection of the compounds of the invention into trees or

plants, it is desirable that the compounds have a relatively high solubility in water, for example a solubility in excess of 30 parts per million. The compounds may be used as an aqueous solution or may be formulated for injection, for example as a solution in a lower alcohol.

For certain applications it is also desirable that the compound has a low persistancy in soil to prevent carry-over to adjacent crops or even crops planted subsequently in the same soil. Preferably the compound for use in such applications has a half life in the soil of less than 20 weeks.

The invention is illustrated by the following Examples in which infra red analysis is given in terms of $\lambda$ max ($cm^{-1}$), NMR analysis is given in terms of $\delta_H$ and mass spectroscopy is given in terms of m/e.

EXAMPLE 1

The preparation of methyl 3-(2,4-dichlorophenyl)-3-hydroxy-2,2-dimethyl-4-(1,2,4-triazol-1-yl)-butanoate (Compound No 1 of Table I).

A solution of 2,4-dichloro-2-(1,2,4-triazol-1-yl)-acetophenone (5.89 g) in dry dichloromethane (150 $cm^3$) was stirred at -78°C under argon, and titanium tetrachloride (2.74 $cm^3$) in dry dichloromethane (10 $cm^3$) was added over 5 minutes, followed by further stirring for 15 minutes at -78°C.

A solution of 1-trimethylsilyloxy-1-methoxy-2-methyl-propene (5 $cm^3$) in dry dichloromethane (10 $cm^3$) was added over 15 minutes. The reaction mixture was then stirred at -78°C for 4 hours. Aqueous potassium carbonate (5%, 150 $cm^3$) was added followed by water (100 $cm^3$). The

resulting slurry was filtered to remove titanium compounds, and the filter bed was washed with dichloromethane (100 $cm^3$). The organic layer of the filtrate was removed. The aqueous layer was extracted with dichloromethane (2 x 100 $cm^3$), and all three organic extracts combined, washed with water until neutral, dried over anhydrous magnesium sulphate and finally filtered. Concentration in vacuo gave a colourless solid. This solid was purified by Flash Chromatography on silica. The product was contained in the first fraction to be eluted with ethyl acetate. The product was dissolved in dichloromethane treated with activated charcoal and filtered. Concentration in vacuo gave 5.1 g (62%) of a colourless solid.

M P t 118.5°C - 119.5°C

Infra red 3100 $cm^{-1}$, 3050 $cm^{-1}$, 1720 $cm^{-1}$

NMR 1.28 (6H,d), 3.7 (3H,s), 5.3 (2H, AB qt), 5.85 (1H, broads), 7.2 (2H, m), 8.18, 7.73 (2H, 2s), 7.85 (1H broad d).

| Microanalysis | C | H | N | |
|---|---|---|---|---|
| | 50.29 | 4.78 | 11.73 | Expected |
| | 50.49 | 4.61 | 11.69 | Found |

EXAMPLE 2

This Example illustrates the preparation of methyl 3-(2,4-dichlorophenyl)-3-hydroxy-2,2-diethyl-4(1,2,4-triazol-1-yl)-butanoate (Compound No 2 of Table 1).

To a solution of 2,4'-dichloro-2-(1,2,4-triazol-1-yl)-acetophenone (2.53 g, 9.9 mmol) in dry dichloromethane (5

ml per mmol) at -78°C and under an inert atmosphere was added a solution of titanium tetrachloride (1.88 g, 9.9 mmol) in dry dichloromethane (1 ml per mmol). After 15 minutes, a solution of 2-ethyl-1-methoxy-1-trimethylsiloxybut-1-ene (2 g, 9.9 mmol) in dry dichloromethane (1 ml per mmol) was added and stirred for 4 hours. The reaction was quenched by the addition of potassium carbonate solution and then poured into water. The inorganic salts were filtered offf and washed with dichloromethane (2 x 100 ml). The organics were separated and the aqueous layer was extracted with dichloromethane (2 x 100 ml). The combined organics were washed with water, dried over magnesium sulphate and the solvent removed. Flash chromatography (silica gel, petrol/ethyl acetate elution) of the residue gave the product (1.55 g, 41%) as a white solid, melting point 96-97°C.

Analysis Found: C, 53.3; H, 5.5; N, 10.7

$C_{17}H_{21}Cl_2N_3O_3$ requires C, 52.9; H, 5.5; N, 10.9%);

Infra red 3200, 3100, 2900, 1750, 1450, 1370, 1230, 1130, 1080, 1020, 880, 860, 800, 720, 600 $cm^{-1}$;

NMR (90 MHz; $CDCl_3$) 0.72 (3H, t, *J*=7.2 Hz); 0.88 (3H, t, *J*=7.2Hz), 1.80 (2H, q, *J*=7.2 Hz), 2.12 (2H,q, *J*=7.2 Hz), 3.70 (3H,s), 4.72 (1H,d, *J*=14.4 Hz), 5.76 (1H,s), 5.98 (1H,d, *J*=14.4 Hz), 7.12 (1H,d, *J*=9 Hz), 7.18 (1H,s), 7.70 (1H,s), 7.83 (1H,d, *J*=9 Hz), 8.12 (1H,s);

| | |
|---|---|
| m/e | 386 ($M+H^+$), 354, 303, 271, 256, 214, 173. |

EXAMPLE 3

This Example illustrates the preparation of ethyl 3-(2,4-dichlorophenyl)-3-hydroxy-2,2-diethyl-4(1,2,4-triazol-1-yl)-butanoate (Compound No 3 of Table I).

Using the general method of Example 2, 2',4'-dichloro-2-(1,2,4-triazol-1-yl)acetophenone (2.53 g, 9.9 mmol) and 1-ethoxy-2-ethyl-1-trimethylsiloxybut-1-ene (2.14 g, 9.9 mmol) were reacted in the presence of titanium tetrachloride (1.88 g, 9.9 mmol) to give the title product (0.9 g, 23%) as a white solid melting point 116-117°C.

| | |
|---|---|
| Analysis: | Found: C, 54.1; H, 5.8; N, 10.3. |
| | $C_{18}H_{23}Cl_2N_3O_3$ requires C, 54.0; H, 5.8; N, 10.5%); |
| Infra red | 3150, 3100, 2900, 1710, 1580, 1500, 1450, 1360, 1270, 1230, 1200, 1130, 1080, 1020, 960, 880, 860, 820, 800, 720, 670, 600 $cm^{-1}$; |
| NMR (90 MHz; $CDCl_3$) | 0.73 (3H, t, *J*=7.2 Hz), 0.88 (3H, t, *J*=7.2 Hz), 1.24 (3H, t, *J*=5.4 Hz), 1.82 (2H, q, *J*=7.2 Hz), 2.12 (2H, q, *J*=7.2 Hz), 4.20 (2H, q, *J*=5.4 Hz), 4.75 (1H, d, *J*=14.4 |

Hz), 5.76 (1H,s), 6.0 (1H, d, J=14.4 Hz), 7.14 (1H, d, J=9 Hz), 7.18 (1H,s), 7.71 (1H,s), 7.88 (1H, d, J=9 Hz) 8.14 (1H,s);

m/e 399 ($M^+$), 317, 256, 214, 158 (100%).

EXAMPLE 4

This Example illustrates the preparation of 1-(1,2,4-triazol-1-yl)-2-(2,4-dichlorophenyl)-2-hydroxy-2-(1-methoxycarbonyl-cyclopent-1-yl) (Compound No 4 of Table I) having the formula:-

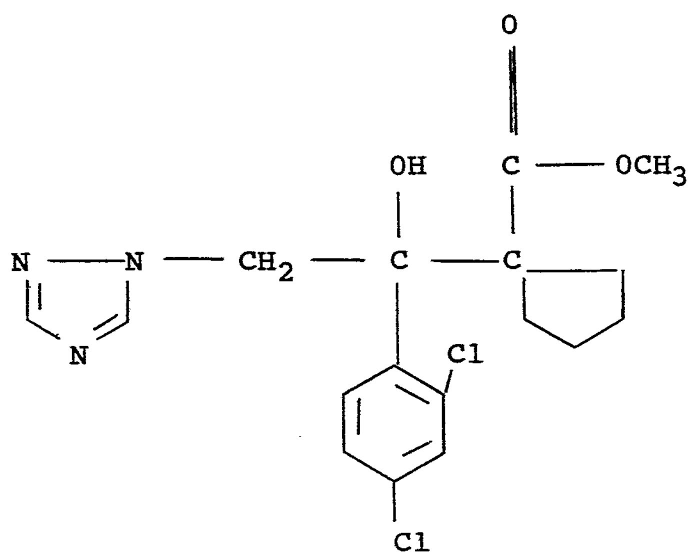

Using the general method of Example 2, 2',4'-dichloro-2-(1,2,4-triazol-1-yl)acetophenone (2.53 g, 9.9 mmol) was reacted with 1-(1-methoxy-1-trimethylsiloxymethylidene)cyclopentane (1.98 g, 9.9 mmol) in the presence of titanium tetrachloride (1.88 g, 9.9 mmol). The product (1.52 g, 40%) was a white solid melting point 114-115.5°C.

| | |
|---|---|
| Analysis | Found: C, 52.5; H 5.1; N, 10.8. |
| | $C_{17}H_{19}Cl_2N_3O_3$ requires C, 53.1; H, 5.0; N, 10.9%); |
| Infra red | 3150, 3100, 2900, 1715, 1590, 1450, 1370, 1270, 1200, 1160, 1090, 1060, 1020, 920, 800, 670 $^{-1}$ cm; |
| NMR (90 MHz; $CDCl_3$) | 1.3-2.3 (8H,m), 3.70 (3H,s) 4.54 (1H, d, J=14.4 Hz), 5.8-6.0 (1H,d, J=6.3 Hz), 7.16 (1H,s), 7.64 (1H,s), 7.85 (1H, d, J=6.3 Hz), 8.17 (1H,s); |
| m/e | 383 ($M^+$), 316, 256, 173 (100%). |

EXAMPLE 5

This Example illustrates the preparation of 1-(1,2,4-triazol-1-yl)-2-(2,4-dichlorophenyl)-2-hydroxy-2-(1-ethoxycarbonyl-cyclobut-1-yl) (Compound No 5 of Table I). Using the general method of Example 2, 2',4'-dichloro-2-(1,2,4-triazol-1-yl)acetophenone (2.53 g, 9.9 mmol) and (1-(1-ethoxy-1-trimethylsiloxymethylidene)cyclobutane

(1.98 g, 9.9 mmol) were reacted in the presence of titanium tetrachloride (1.88 g, 9.9 mmol). The product (1.13 g 30%) was a white solid melting point 100-102°C.

| | |
|---|---|
| Analysis | Found: C, 52.3; H, 5.0; N, 10.7. |
| | $C_{17}H_{19}Cl_2N_3O_3$ requires C, 53.1; H, 5.0; N, 10.9%); |
| Infra red | 3150, 3100, 2900, 1710, 1590, 1450, 1370, 1290, 1200, 1140, 1070, 1020, 800, 720, 600 $cm^{-1}$; |
| NMR (90 MHz; $CDCl_3$) | 1.14, (3H, t, J=6.3 Hz), 1.6-2.8 (6H,m), 4.11 (2H, q, J=6.3 Hz), 4.54 (1H, d, J=14.4 Hz), 5.32 (1H,s), 5.62 (1H, d, J= 14.4 Hz), 7.1-7.28 (2H,m) 7.66 (1H, d, J=7.2 Hz), 7.76 (1H,s), 8.14 (1H,s); |
| m/e | 384 ($M+H^+$), 383, 256, 214 (100%), 173. |

EXAMPLE 6

This Example illustrates the preparation of 1-(1,2,4-triazol-1-yl)-2-(4-trifluoromethylphenyl)-2-hydroxy-2-(1-methoxycarbonyl-cyclopent-1-yl) (Compound No 6 of Table I). Using the general method of Example 2, 2-(1,2,4-triazol-1-yl)-4'-trifluoromethylacetophenone (2.53 g, 9.9 mmol) and 1-(1-methoxy-1-trimethylsiloxymethylidene)cyclopentane (1.98 g, 9.9 mmol) were reacted in the presence of titanium tetrachloride

(1.88 g, 9.9 mmol). The product (1.71 g, 45%) was a pale yellow gum.

| | |
|---|---|
| Analysis | Found: C, 55.0; H, 5.5; N, 10.6. |
| | $C_{18}H_{20}F_3N_3O_3$ requres C, 56.4; H, 5.3; N, 11.0%); |
| Infra red | 3350, 3100, 2950, 1700, 1610, 1500, 1400, 1320, 1270, 1160, 1110, 1060, 1000, 950, 920, 825, 825, 720, 670, 650, 610 $cm^{-1}$; |
| NMR (90 MHz; $CDCl_3$) | 1.4-2.2 (8H,M), 3.64 (3H,s), 4.70 (1H, d, *J*=14.4 Hz), 5.25 (1H, d, *J*=14.4 Hz), 5.52 (1H,s), 7.47 (4H,s), 7.74 (1H,s), 7.98 (1H,s); |
| m/e | 384 ($M+H^+$), 364, 352, 301, 256, 145, 83 (100%). |

EXAMPLE 7

This Example illustrates the preparation of methyl 3-(4-trifluoromethylphenyl)-3-hydroxy-2,2-dimethyl-4-(1,2,4-triazol-1-yl)-butanoate. (Compound No 7 of Table I). Using the general method of Example 2, 2-(1,2,4-triazol-1-yl)-4'-trifluoromethylacetophenone (2.53 g, 9.9 mmol) and 1-methoxy-2-methyl-1-trimethylsiloxypropene (1.73 g, 9.9 mmol) were reacted in the presence of titanium tetrachloride (1.88 g, 9.9 mmol). The product (1.61 g, 465) was a white solid melting point 98-99°C.

| | |
|---|---|
| Analysis | Found: C, 53.4; H, 5.0; N, 11.7. |
| | $C_{16}H_{18}F_3N_3O_3$ requires C, 53.8; H, 5.1; N, 11.8%); |
| Infra red | 3150, 3100, 2900, 1700, 1605, 1510, 1450, 1370, 1320, 1280, 1200, 1150, 1110, 1080, 1060, 970, 840, 715, 675, 620 $cm^{-1}$; |
| NMR (MHz; $CDCl_3$) | 1.23 (3H,s), 1.27 (3H,s), 3.68 (3H,s), 4.98 (2H,dd, J=14.4 and 40 Hz), 5.56 (1H, brs), 7.51 (4H,s), 7.74 (1H,s), 7.98 (1H,s); |
| m/e | 338 ($M^+$-F), 326, 275, 256, 173 (100%), 145. |

EXAMPLE 8

This Example illustrates the preparation of methyl 3-(2,4-dichlorophenyl)-3-hydroxy-2-methyl-2-ethyl-4-(1,2,4-triazol-1-yl)-butanoate. (Compound No 8 of Table I). Using the general method of Example 2, 2',4'-dichloro-2-(1,2,4-triazol-1-yl)acetophenone (2.53 g, 9.9 mmol) and 1-methoxy-2-methyl-1-trimethylsiloxybut-1-ene (1.86 g, 9.9 mmol) were reacted in the presence of tatanium tetrachloride 91.88 g, 9.9 mmol). The product, a mixture of two isomers, (1.98 g, 54%) was pale yellow solid melting point 70.5-71.5°C.

| | |
|---|---|
| Analysis | Found: C, 50.8; H, 4.9; N, 10.8. |

$C_{16}H_{19}Cl_2N_3O_3$ requires C, 51.6; H, 5.1; N, 11.3%);

| | |
|---|---|
| Infra red | 3150, 3100, 2900, 1710, 1580, 1450, 1370, 1230, 1140, 1080, 1020, 845, 880, 800, 730, 675, 600 $cm^{-1}$; |
| NMR (90 MHz; $CDCl_3$) | 0.82 (2H,t, *J*=7.2 Hz), 0.84 (3H,t, *J*=7.2 Hz), 1.14 (3H,s), 1.19 (3H,s), 1.5-2.36 (4H,M), 3.74 (3H,s), 3.75 (3H,s), 4.65 (1H, d, *J*=13.5 Hz), 4.80 (1H,d, *J*=13.5 Hz), 5.31 (1H,s), 5.80 (1H,s), 6.0 (1H,d, *J*=13.5 Hz), 6.02 (1H, d, *J*=13.5 Hz), 7.08-7.28 (4H,m), 7.74 (2H,s), 7.87 (2H, d, *J*=8.1 Hz), 8.17 (1H,s), 8.21 (1H,s); |
| m/e | 372 ($M+H^+$), 304, 256, 214, 173 (100%). |

The manner in which the compounds of the present invention may be formulated into compositions suitable for application is shown generally in the following indicative illustrations numbered as Examples 9 to 18.

## EXAMPLE 9

An emulsifiable concentrate is made up by mixing the following ingredients, and stirring the mixture until all the constituents were dissolved.

| | |
|---|---|
| Compound of invention | 10% |
| Calcium dodecylbenzensulphate | 5% |
| "SYNPERONIC" NP13 | 5% |
| "Aromasol" H | 80% |

EXAMPLE 10

A composition in the form of grains readily dispersible in a liquid, eg, water, is prepared by grinding together the first three ingredients in the presence of added water and then mixing in the sodium acetate. The resultant mixture is dried and passed through a British Standard mesh sieve, size 44-100, to obtain the desired size of grains.

| | |
|---|---|
| Compound of invention | 50% |
| "Dispersol" T | 25% |
| 'SYNPERONIC" NP5 | 1.5% |
| Sodium acetate | 23.5% |

EXAMPLE 11

The following ingredients are ground together to produce a powder formulation readily dispersible in liquids.

| | |
|---|---|
| Compound of invention | 45% |
| "Dispersol" T | 5% |
| "SYNPERONIC" NX | 0.5% |
| "Cellofas" B600 | 2% |
| China clay GTY powder | 47.5% |

EXAMPLE 12

The active ingredient is dissolved in acetone and the resultant liquid is sprayed on to the granules of attapulgite clay. The solvent is then allowed to evaporate to produce a granular composition.

| | |
|---|---|
| Compound of invention | 5% |
| Attapulgite granules | 95% |

EXAMPLE 13

A composition suitable for use as a seed dressing is prepared by mixing the three ingredients.

| | |
|---|---|
| Compound of invention | 50% |
| Mineral oil | 2% |
| China clay | 48% |

EXAMPLE 14

A dusting powder is prepared by mixing the active ingredient with talc.

| | |
|---|---|
| Compound of invention | 5% |
| Talc | 95% |

EXAMPLE 15

A flowable formulation is prepared by bead-milling the constituents set out below and then forming an aqueous suspension of the ground mixture with water.

| | |
|---|---|
| Compound of invention | 40% |
| "Dispersol" T | 4% |
| "SYNPERONIC" NP5 | 1% |
| Water | 55% |

EXAMPLE 16

A dispersible powder formulation is made by mixing together the ingredients set out below and then grinding the mixture until all were thoroughly mixed.

| | |
|---|---|
| Compound of invention | 25% |
| "Aerosol" OT/B | 2% |
| "Dispersol" A.C. | 5% |
| China clay | 28% |
| Silica | 40% |

EXAMPLE 17

This Example illustrates the preparation of a dispersible powder formulation. The ingredients are mixed and the mixture then ground in a comminution mill.

| | |
|---|---|
| Compound of invention | 25% |
| "PERMINAL" BX | 1% |
| "Dispersol" T | 5% |
| Polyvinylpyrrolidone | 10% |
| Silica | 25% |
| China clay | 34% |

EXAMPLE 18

The ingredients set out below are formulated into dispersible powder by mixing then grinding the ingredients.

| | |
|---|---|
| Compound of invention | 25% |
| "Aerosol" OT/B | 2% |
| "Dispersol" A | 5% |
| China clay | 68% |

In Examples 9 to 18 the proportions of the ingredients given are by weight.

There now follows an explanation of the compositions or substances represented by the various Trade Marks and Trade Names mentioned above.

| | |
|---|---|
| "SYNPERONIC" NP13 : | a condensate of nonyl phenol (1 mole) with ethylene oxide (13 moles). |
| "AROMASOL" H : | a solvent mixture of alkylbenzenes. |
| "DISPERSOL" T AND AC : | a mixture of sodium sulphate and a condensate of formaldehyde with sodium naphthalene sulphonate. |
| "SYNPERONIC" NP5 | a condensate of nonyl phenol (1 mole) with naphthalene oxide (5.5 moles). |
| CELLOFAS B600 : | a sodium carboxymethyl cellulose thickener. |

## EXAMPLE 19

### Whole Plant Screen

The compound of Example 1 (Compound No 1 of Table I) was tested on a whole plant screen. The compound was tested for plant growth regulator activity against up to eleven species for various growth effects relevant to plant growth regulation.

### Methodology

The plant species used in this screen are presented in Table II with the leaf stage at which they were sprayed. Each chemical was applied at 4000 ppm (4kg/ha in a 1000 l/ha field volume) using a tracksprayer and a SS8004E (Teejet) nozzle.

After spray the plants were grown in a glasshouse with 25°C day/22°C night temperatures. The exception to this were the temperature cereals, wheat and barley which are grown in 13-16°C day/11-13°C night temperatures. Supplementary lighting was supplied when necessary to provide an average photoperiod of 16 hours (14 hours minimum).

After 2-6 weeks in the glasshouse, depending on species and time of year, the plants were visually assessed for morphological characteristics. Formulation blanks were used as controls to assess the plants against. The results are presented in Table III.

TABLE II

PLANT MATERIAL USED FOR ALTERNATIVE WHOLE PLANT SCREEN

| Species | Code | Variety | Growth Stage at Treatment | No. Plants per 3" Pot | Compost Type |
| --- | --- | --- | --- | --- | --- |
| Barley | BR | Atem | 1 - 1.5 leaves | 4 | JIP* or PEAT |
| Wheat | WW | Timmo | 1 - 1.5 leaves | 4 | JIP or PEAT |
| Maize | MZ | Earliking | $2\frac{1}{4}$ - $2\frac{1}{2}$ leaves | 1 | PEAT |
| Vines | VN | Ohanez + Unspecified | 4 leaves | 1 | PEAT |
| Soya | SY | Amsoy | 1st trifoliate | 1 | JIP |
| Tomato | TO | Ailsa Craig | 1.5 - 2 leaves | 1 | PEAT |
| Lettuce | LT | Verpia | 3 - 4 leaves | 1 | PEAT |
| Sugar Beet | SB | Amono | 2 leaves | 1 | PEAT |
| _Agrostis tenius_ | AT | | cut to 2 cm | Grown in rows | |
| _Cynosurus cristatus_ | CC | | 48 hours before | in plastic | PEAT |
| _Dacrylis glomerata_ | DA | | treatment | punnets | |

JIP* = John Innes Potting Compost.

TABLE III

| COMPOUND NO | WW | BR | MZ | AT | CC | DA | SY | SB | TO | VN | LT |
|---|---|---|---|---|---|---|---|---|---|---|---|
| 1 | 3G | 2G | | 1 | 2 | 2 | 2GA | 2GA | 3GA | 3GA | |

KEY

Retardation 1-3 where 1 = 10-30% Retardation
2 = 21-60% Retardation
3 = 61-100% Retardation

Greening effect = G
Apical damage = A
Tillering or side shooting = T

Blank means less than 10% effect

- indicates that the compound was not tested against this species

EXAMPLE 20

Whole Plant Screen

Compound numbers 2 to 5 were tested on an alternative whole plant screen. The compound were tested for plant growth regulator activity against five species for various growth effects relevant to plant growth regulation.

Methodology

The plant species used in this screen are presented in Table IV with the leaf stage at which they were sprayed. Each chemical was applied at 4000 ppm (4 kg/ha in a 1000 l/ha field volume) using a tracksprayer and a SS8004E (Teejet) nozzle.

After spray the plants were grown in a glasshouse with 25°C day/22°C night temperatures. The exception to this were the temperature cereals, wheat and barley which are grown in 13-16°C day/11-13°C night temperatures. Supplementary lighting was supplied when necessary to provide an average photoperiod of 16 hours (14 hours minimum).

After 2-6 weeks in the glasshouse, depending on species and time of year, the plants were visually assessed for morphological characteristics. Formulation blanks were used as controls to assess the plants against. The results are presented in Table V.

TABLE IV

PLANT MATERIAL USED FOR ALTERNATIVE WHOLE PLANT SCREEN

| Species | Code | Variety | Growth Stage at Treatment | No Plants per 3" Pot | Compost Type |
|---|---|---|---|---|---|
| Barley | BR | Atem | 1 - 1.5 leaves | 4 | JIP* |
| Wheat | WW | Timmo | 1 - 1.5 leaves | 4 | JIP |
| Maize | MZ | Earliking | $2\frac{1}{4}$ - $2\frac{1}{2}$ leaves | 1 | PEAT |
| Apple | AP | Red Delicious | 4 - 5 leaves | 1 | JIP |
| Rice | RC | Ishikari | 2 - $2\frac{1}{2}$ leaves | 4 | JIP |

JIP* = John Innes Potting Compost.

TABLE V

| SPECIES | COMPOUND NO | R | G | A | T | I |
|---|---|---|---|---|---|---|
| BR | 2 | 2 | | | | 2 |
| | 3 | 2 | 2 | | 2 | 3 |
| | 4 | 1 | | | | 1 |
| | 5 | 3 | 2 | | | 3 |
| WW | 2 | 1 | | | | 1 |
| | 3 | 3 | 2 | | | 3 |
| | 4 | 1 | | | | 2 |
| | 5 | 3 | | | | 3 |
| RC | 2 | | | | | |

TABLE V CONTINUED

| SPECIES | COMPOUND NO | R | G | A | T | I |
|---|---|---|---|---|---|---|
| | 3 | | | | | |
| | 4 | 1 | | | | 2 |
| | 5 | 3 | 1 | | 1 | 3 |
| MZ | 2 | 2 | 1 | 1 | 1 | 3 |
| | 3 | 1 | | | | |
| | 4 | | | | | |
| | 5 | 1 | | 2 | | |
| AP | 2 | 3 | 2 | 3 | | |
| | 3 | 3 | 2 | 3 | | |

TABLE V CONTINUED

| SPECIES | COMPOUND NO | R | G | A | T | I |
|---|---|---|---|---|---|---|
| | 4 | 3 | 2 | 3 | | |
| | 5 | 3 | 2 | 3 | | |

KEY

R = Retardation

G = Greening effect

A = Apical damage

T = Tillering or side shooting

I = Interligular or internodal length reduction

All effects are scored visually on a 1-3 basis where

1 = 10-30%

2 = 21-60%

3 = 61-100%

Blank means less than 10% effect

EXAMPLE 21

Intermediate Retardant Test

Methodology

Two species are involved in this test RICE and SPRING-BARLEY. The variety and growth stages at spray are outlined in Table VI. Compounds were applied at rates from 50 to 4000 ppm (0.05 upto 4kg $ha^{-1}$ at a field volume of 1000 $ha^{-1}$) as an overall spray. This gives a foliar and root component in the test, ie, this test will detect the activity of both root and foliar acting compounds. The rice was grown in 4" 'paddy' pots, ie, the roots and bottom of the stems are immersed in water under conditions corresponding to those in paddy fields. Spring barley was grown in 4" pots. The plants were assessed for height to top-most ligule at aproximately 28 days after treatment. The results are presented in Tables VII and VIII. In eachcase the result is presented as a percentage reduction in height compared to the formulation blank.

Compound 1 in the Tables is the compound of Example 1. A blank indicates that no retardant effect was observed.

TABLE VI

PLANT MATERIAL FOR INTERMEDIATE RETARDANT TEST

| Species | Variety | Growth Stage at Treatment | No. Plants per 4" Pot | Compost Type |
|---|---|---|---|---|
| Spring Barley | Atem | 3 leaves | 4 | JIP 1 |
| Rice | Ishikari | 3 - 4 leaves | 2 | SM2 : JIP 1 |

JIP 1 = John Innes Potting Compost.

SM2 = a mixture of loam and grit.

TABLE VII

Percentage Reduction in Height of Rice
(Compound to formulation blank)

| COMPOUND NO | Rate (ppm) | | | | | |
|---|---|---|---|---|---|---|
| | 4000 | 1000 | 500 | 250 | 100 | 50 |
| 1 | 40.1 | 47.2 | 52.3 | 47.6 | 38.5 | 20.2 |

TABLE VIII

Percentage Reduction in Height of Spring Barley
(Compared to formulation blank)

| COMPOUND NO | Rate (ppm) | | |
|---|---|---|---|
| | 4000 | 1000 | 500 |
| 1 | 77.6 | 22.4 | 8.5 |

EXAMPLE 22

This Example compares Compound No 1 of Table I of the present invention with prior art Compound No 9 of Table I of EP 0046633, whose structure is:-

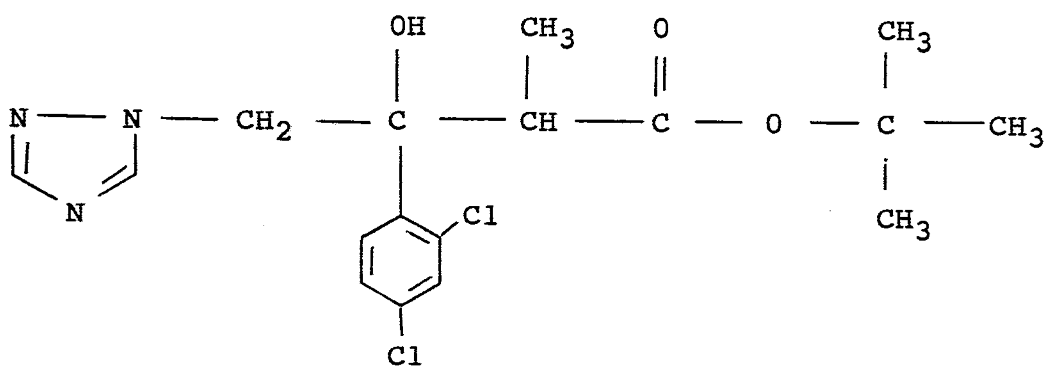

The compounds were evaluated in side-by-side tests against the species indicated in Table VI using the general method of Example 21. The results are given in Tables IX and X which indicate the marked and suprising advantage of the compound of the present invention, especially in respect of rice.

TABLE IX

Percentage Reduction in Height of Spring Barley
(Compared to formulation blank)

| COMPOUND NO | Rate (ppm) | |
|---|---|---|
| | 2000 | 500 |
| 1 | 37 | 11 |
| 9 of EP 00046633 | 21 | 8 |

TABLE X

Percentage Reduction in Height of Rice
(Compared to formulation blank)

| COMPOUND NO | Rate (ppm) | |
|---|---|---|
| | 2000 | 500 |
| 1 | 42 | 22 |
| 9 of EP 00046633 | 9 | 4 |

MJR/CF
PP33739
15 Dec 86

CLAIMS

1. A plant growth regulating compostion comprising a triazole derivative having the general formula (I) and stereoisomers thereof and a carrier or diluent:-

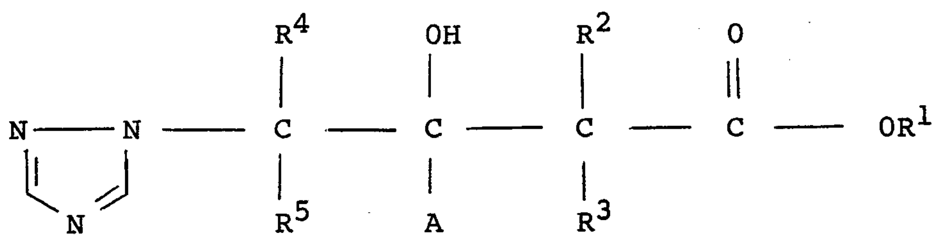

(I)

wherein $R^1$ is an alkyl group containing from 1 to 4 carbon atoms; $R^2$ and $R^3$, which may be the same or different, are each separately an alkyl group containing from 1 to 4 carbon atoms, or $R^2$ and $R^3$, together with the carbon atom joining them, from a cycloalkyl group containing from 3 to 6 carbon atoms; $R^4$ and $R^5$, which may be the same or different, each represent hydrogen or an alkyl group containing from 1 to 4 carbon atoms; and A represents an optionally substituted phenyl group; and acid addition salts, ethers, esters and metal complexes thereof.

2. A composition according to claim 1 wherein $R^1$ is methyl, ethyl or propyl.

3. A composition according to claim 1 or 2 wherein $R^2$ and $R^3$, which may be the same or different, are methyl or ethyl or, together with the carbon atoms joining them, from a cycloalkyl group which is cyclobutyl or cyclopentyl.

4. A composition according to any of the preceding claims wherein $R^4$ and $R^5$ are hydrogen or methyl.

5. A composition according to any of the preceding claims wherein A is phenyl substituted by one or more of halogen, trifluoromethyl or alkyl containing from one to 4 carbon atoms.

6. A composition according to claim 5 wherein A is substituted at the 4-position or at the 2,4-positions.

7. A composition according to claim 6 wherein A is 2,4-dichlorophenyl or 4-trifluoromethylphenyl.

8. A triazole derivative of formula (I).

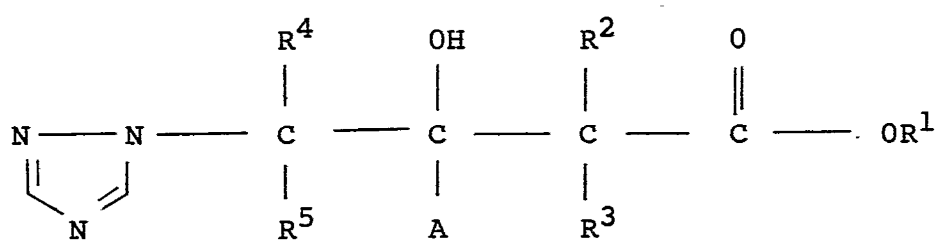

(I)

and stereoisomers thereof, wherein $R^1$ is an alkyl group containing from 1 to 4 carbon atoms; $R^2$ is an alkyl group containing from 1 to 4 carbon atoms, $R^3$ is an alkyl group containing from 2 to 4 carbon atoms, or $R^2$ and $R^3$, together with the carbon atom joining them, form a cycloalkyl group containing from 3 to 6 carbon atoms; $R^4$ and $R^5$, which may be the same or different, each represent hydrogen or an alkyl group containing

from 1 to 4 carbon atoms; and A represents an optionally substituted phenyl group; and acid addition salts, ethers, esters and metal complexes thereof.

9. A triazole derivative according to claim 8 wherein $R^1$ is methyl, ethyl or propyl.

10. A triazole derivative according to claim 8 or 9 wherein $R^2$ is ethyl and $R^3$ is methyl or ethyl or $R^2$ and $R^3$, together with the carbon atoms joining them, form a cycloalkyl group which is cyclobutyl or cyclopentyl.

11. A triazole derivative according to any of claims 8 to 10 wherein $R^4$ and $R^5$ are hydrogen or methyl.

12. A triazole derivative according to any of claims 8 to 11 wherein A is phenyl substituted by one or more of halogen, trifluoromethyl or alkyl containing from 1 to 4 carbon atoms.

13. A triazole derivative according to claim 12 wherein A is substituted at the 4-position or at the 2,4-positions.

14. A triazole derivative according to claim 13 wherein A is 2,4-dichlorophenyl or 4-trifluoromethylphenyl.

15. Methyl 3-(2,4-dichlorophenyl)-3-hydroxy-2,2-dimethyl-4(1,2,4-triazol-1-yl)-butanoate.

16. A process for preparing a compound of formula (I) in claim 1 which comprises reacting a compound of formula (II), wherein $R^4$, $R^5$ and A have the meanings given in claim 1 or claim 8, with a compound of general formula

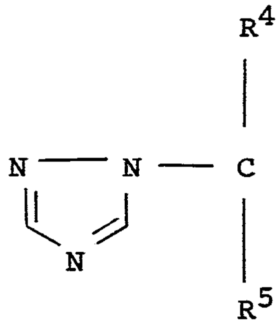

(II)

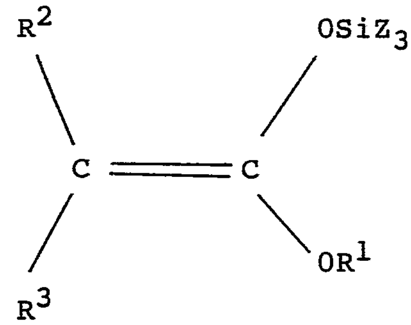

(III)

(III) wherein $R^1$, $R^2$ and $R^3$ have the meanings given in claim 1 and Z is an alkyl or aryl group; in the presence of a Lewis acid.

17. A process according to claim 16 wherein the Lewis acid is titanium tetrachloride.

18. A method of regulating the growth of plants which comprises applying to the plant, to the seed of the plant, or the locus of the plant or seed a triazole derivative of general formula (I) in claim 1.

European Patent Office

# EUROPEAN SEARCH REPORT

0 235 877

EP 87 30 0106

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 060 223 (CIBA-GEIGY AG) * table I, compounds nos. 1.19, 1.191 in combination with page 16, lines 1-17, examples 7, 9, page 40, lines 5, 6 * | 8-15 | C 07 D 249/08<br>A 01 N 43/653 |
| Y | * page 24, lines 1-3, table IV, compound no. 4.58 * | 1-7,18 | |
| | --- | | |
| Y,D | EP-A-0 046 633 (ICI PLC) * page 1, lines 1-16, table I, compounds 9, 11, claims 8-10 * | 1-7,18 | |
| | --- | | |
| X | CHEMICAL ABSTRACTS, vol. 84, no. 3, 19th January 1976, page 485, column 2, abstract no. 17469s, Columbus, Ohio, US; K. SAIGO et al.: "New method for the preparation of beta-hydroxyesters. Titanium tetrachloride-promoted reaction of ketene alkyl trialkylsilyl acetals with carbonyl compounds", & CHEM. LETT. 1975, (9), 989-990 | 16,17 | TECHNICAL FIELDS SEARCHED (Int. Cl.4)<br>C 07 D 249/00<br>A 01 N 43/00<br>C 07 D 521/00 |
| | --- | | |
| Y,D | EP-A-0 106 515 (PFIZER LTD.) * page 2, line 1 - page 3, line 10, page 15, formula VIII, example 41 * | 1-7,18 | |
| | --- -/- | | |

The present search report has been drawn up for all claims

| Place of search | Date of completion of the search | Examiner |
|---|---|---|
| BERLIN | 10-04-1987 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82

European Patent Office

**EUROPEAN SEARCH REPORT**

**0 235 877**

EP 87 30 0106

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 055 833 (BAYER AG)<br>* claims 1, 4-10, in particular page 89, lines 13-15 *<br>----- | 1-7,18 | |
| | | | TECHNICAL FIELDS SEARCHED (Int. Cl.4) |

The present search report has been drawn up for all claims

| Place of search | Date of completion of the search | Examiner |
|---|---|---|
| BERLIN | 10-04-1987 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document